# EUROPEAN PATENT SPECIFICATION

(11) **EP 3 847 662 B1**
(45) Date of publication and mention of the grant of the patent: **15.01.2025**
(21) Application number: 19762954.6
(22) Date of filing: 03.09.2019
(51) Int. Cl.: G16H 40/63, G16H 50/30

(54) **METHOD AND ARRANGEMENT FOR RESPIRATORY MEASUREMENT**
VERFAHREN UND SYSTEM ZUR ATEMMESSUNG
PROCÉDÉ ET AGENCEMENT DE MESURE RESPIRATOIRE

(30) Priority: 07.09.2018 US 201862728268 P
(43) Date of publication of application: 14.07.2021
(73) Proprietor: Revenio Research Oy, 01510 Vantaa (FI)
(72) Inventor: SEPPÄ, Ville-Pekka, 01510 Vantaa (FI); HULT, Anton, 01510 Vantaa (FI)
(74) Representative: Heinonen & Co
(86) International application number: PCT/EP2019/073461
(87) International publication number: WO 2020/048978

(56) References cited:
- US-A1- 2014 032 120
- US-A1- 2016 135 715

## Description

### FIELD OF THE INVENTION

Generally, the present invention relates to respiratory measurement. Particularly, the present invention pertains to a method, a computer program product and an arrangement for measuring and detecting changes in human respiration.

### BACKGROUND

Lung function measurement is the cornerstone of monitoring and diagnosing of a plurality of lung diseases. However, subjects with limited co-operation capability due to their developmental phase or mental or physical limitations are not capable of performing normal lung function tests that require demanding respiratory maneuvers. For instance, diagnosis of asthma in preschool children is difficult because of unsuitability of the conventional lung function testing.

Measurements during spontaneous tidal breathing (TB) require minimal co-operation, thus being suitable for small children and infants. There is a large body of research suggesting that parameters derived from TB flow curves or flow-volume (TBFV) curves change in a deterministic way with obstructive respiratory diseases in young patients. The studies have shown for instance that TB parameters relate to forced expiratory volume in 1 second (FEV1), airway resistance, bronchodilator response, and methacholine challenge and that they can be used to discriminate between pathological respiratory conditions.

The current techniques and arrangements for measuring and analyzing the TB pattern are hindered by the need of a direct access with the airways. Sedation can sometimes be used to overcome the psychological aspects of the measurement, but the physical face contact and the increased dead space still distort the respiratory pattern. Especially analysis of temporal variability of tidal breathing would benefit from longer TB recordings that are not feasible with instruments requiring direct airway access.

US2014032120 Al discloses methods of extracting optimal information from a flow/volume loop concerning the patient's respiratory condition by examining the overall shape of the space under the flow/volume loop's expiration portion based on the area of the expiration space for the first volume half of expiration relative to the area of the expiration space for the second volume half of expiration. An expression pattern is used to refer to one individual shape of a curve representing a single expiration cycle. The document is not concerned with analysing the variability between the expiration phases of multiple respiration cycles based on measurement data in the range of the first half of expired volume in the expiration phases.

### SUMMARY OF THE INVENTION

The objective of the embodiments of the present invention is to at least alleviate one or more of the aforementioned drawbacks evident in the prior art arrangements particularly in the context of methods and arrangements for respiratory measurement. The objective is generally achieved with a method, arrangement and computer program product in accordance with the present disclosure.

An advantage of the present invention is that it allows for measuring a person's respiration in a way which may be used to detect changes in the person's respiration. Such detected changes in respiration may be afterwards used to make diagnoses of the causes of the detected changes in respiration.

In accordance with one aspect of the present invention a computer-implemented method for measuring changes in respiration using measurement data representing a plurality of measured respiration cycles in the form of flow and volume of respiration, or flow and time of respiration, or time and volume of respiration, over a duration of time, and wherein such measurement data pertains to at least the expiration phase measurement of the respiration cycles,
analyzing variability of the expiration phases of flow-volume, flow-time or time-volume measurements of the respiration cycles wherein the measurements are measurements over a duration of time,
wherein the variability between the expiration phases of the respiration cycles is analyzed from the measurement data in the range of the first half of expired volume in the expiration phase of the respiration cycles.

In accordance with another aspect of the present invention an arrangement for measuring changes in respiration comprising measuring means for measuring flow and volume of respiration or flow and time of respiration or time and volume of respiration over a duration of time, and wherein such measurement pertains to at least the expiration phase measurement of the respiration cycles,
and further comprising computing means arranged to,
analyze variability of the expiration phases of flow-volume, flow-time or time-volume measurements of the respiration cycles wherein the measurements are measurements measured over a duration of time,
wherein the variability between the expiration phases of the respiration cycles is analyzed from the measurement data in the range of the first half of expired volume in the expiration phase of the respiration cycles.

In accordance with another aspect of the present invention a computer program product embodied in a non-transitory computer read-able medium, comprising computer code for causing the computer to execute the method of claim 1.

As briefly reviewed hereinbefore, the utility of the different aspects of the present invention arises from a plurality of issues depending on each particular embodiment.

The expression "a number of" may herein refer to any positive integer starting from one (1). The expression "a plurality of" may refer to any positive integer starting from two (2), respectively.

The term "exemplary" refers herein to an example or example-like feature, not the sole or only preferable option.

The expression "tidal volume" is the volume representing the volume of air displaced during single normal inspiration or expiration. Consequently, the expression "tidal breathing" is used to refer to such normal breathing wherein the volume is tidal volume.

The expression "respiration cycle" is used to refer to the cycle of breathing including both expiration and inspiration. The expression "expiration phase" is used to refer to expiration of the respiration cycle excluding the inspiration of the respiration cycle.

Different embodiments of the present invention are also disclosed in the attached dependent claims.

### BRIEF DESCRIPTION OF THE DRAWINGS

Some exemplary embodiments of the present invention are reviewed more closely with reference to the attached drawings, wherein
Fig. 1 depicts an embodiment of a measurement means arrangement suitable for the method in accordance with the present invention,
Fig. 2 depicts another embodiment of a measurement means arrangement suitable for the method in accordance with the present invention,
Fig. 3 depicts a flow diagram of an embodiment of the method in accordance with the present invention,
Fig. 4 depicts a diagram illustrating p-values from a Wilcoxon rank-sum test between the variabilities of TBFV measurements of two groups, wherein the first group comprises TBFV measurement data of asthmatic persons and the second group comprise TBFV measurement data of healthy persons,
Fig. 5 depicts a graph illustrating a plurality of flow-volume curves obtained from a person during a continuous measurement during sleep in a timeframe,
Fig. 6 depicts another graph illustrating a plurality of flow-volume curves obtained from a person during a continuous measurement during sleep in a timeframe,
Fig. 7 depicts another graph illustrating a plurality of flow-volume curves obtained from a person during a continuous measurement during sleep in a timeframe.

### DETAILED DESCRIPTION OF THE EMBODIMENTS

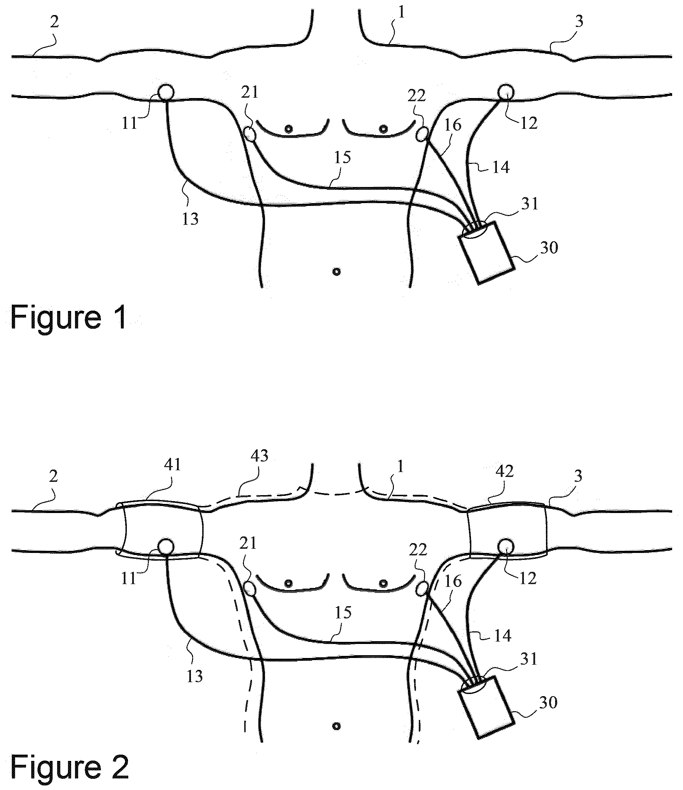

Fig. 1 depicts an embodiment of a measurement means arrangement suitable for the method in accordance with the present invention. An apparatus for impedance pneumograhy **30** is connected via a connector interface **31** to the sensor **11** attached to the right arm **2** and the sensor **12** attached to the left arm **3** of a human body **1.** Sensors **21, 23** are attached to the side of thorax or to the midaxillary line on both sides of the body **1.** The sensor element comprises an electrode and a cable **13, 14, 15, 16** conducting the electrical signal to the connector interface **31.** The midaxillary line is defined as a coronal line on the torso between the anterior axillary line and the posterior axillary line. The sensor placement may vary few centimetres from the midaxillary line.

Sensors **11, 12, 21, 22,** cables, **13, 14, 15, 16,** the interface **31** and the apparatus **30** are components of an impedance pneumography measurement system. The sensors **11, 12, 21, 22** may comprise a text, colour or other indication that helps the person using the impedance pneumography system to connect the sensor to a correct position on the body **1.** Sleeves **41, 41** may comprise an indication separating the left arm **2** and the right arm **3.** Also the sizing or the form of the sleeve **41, 42** may prevent the user from installing the sensor **11, 12** to a wrong position.

In one embodiment of the arrangement the interface **31** configured to the apparatus **30** is arranged to comprise indication of a correct installation procedure, such as colour coding or text. The apparatus **30** may also comprise a display for informing the user about the procedure. The software implemented in the apparatus **30** may also comprise code for providing assistive information to the user, confirming the correct installation procedure or informing about any errors during the installation or operation. One example of an error situation is the measurement data being out of the predefined range.

The apparatus **30** may comprise an interface to transmit the impedance pneumography information to another device, such as a computer or another medical device. In one embodiment the apparatus **30** is arranged to convert changes in thoracic impedance resulting from respiration into a high level respiration signal that can be used with other applications. The apparatus **30** may also be integrated into another medical device.

Fig. 2 depicts another embodiment of a measurement means arrangement suitable for the method in accordance with the present invention where sensors **11, 12** are arranged to be part of a sleeve **41, 42.** The sleeve **41, 42** is made from electrically resistive material that prevents the direct skin contact between the arm **2, 3** and the torso. This prevents the electrical current from passing through the skin and thus contributing to false values. The bioimpedance values are measured through the high-axillary line or from the preferred path of the upper portion of lungs. The sleeve may also be part of a shirt or jacket **43** arranged to be used with the impedance pneumography system. The sleeve may also be in the form of an armband. In one embodiment the thickness of the armband keeps the arm at a distance from the body. The sleeve may also comprise the electrode configured as a fabric electrode made of suitable material such as silver or platinum.

Sensors **11, 12, 21, 22** may be arranged in different configurations. In a tetrapolar bioimpedance measurement four electrodes are used; two for feeding an alternating current of a constant amplitude and two for sensing the voltage. Also a constant voltage may be used while the current is measured. The electrode is measuring for example the voltage differential measured from both arms or the electrodes may be feeding the current to enable measuring of the impedance. The pair of electrodes purposed for the same parameter is always positioned to a distance from each other. Feeding the current and measuring the voltage may also be combined into a single sensor as a pair of electrodes.

In the impedance pneumography a small high frequency current is passed through a pair of skin electrodes and another pair of electrodes is used to record the generated voltage that is proportional to the impedance, which again is proportional to the lung volume. The cardiogenic oscillations can be removed by a filtering technique described in the Finnish patent application FI20115110.

Placing the electrodes **11, 12** on the arms **2, 3** improves significantly the linearity of the measurement results on an impedance to lung volume scale, especially at low lung volumes. One exemplary placement of the electrodes is between biceps and triceps brachii muscles. This placement of the electrodes on the arms can be described as placement on the supraaxillary line. Preventing the skin contact between the arms and the sides improves the measurement as the skin contact is not contributing to the bioimpedance value.

An example of method for filtering cardiogenic oscillations from the thoracic impedance signal is presented herein.

Suppressing an oscillatory signal Sosc is carried out by providing a composite signal S comprising said oscillatory signal Sosc and a modulating signal Smod; high pass filtering the composite signal S with a high pass filter to produce an estimate of the oscillatory signal Sosc and an estimate of the modulating signal Smod, wherein the estimate of the oscillatory signal Sosc comprises first oscillations during a first state of the modulating signal Smod and second oscillations during a second state of the modulating signal Smod; defining a first bin associated with said first state and a second bin associated with said second state; assigning the first bin for said first oscillation according to a state defined from the estimate of the modulating signal Smod and the second bin for said second oscillation according to a state defined from the estimate of the modulating signal Smod; forming a first average waveform for said first oscillations in said first bin and a second average waveform for said second oscillations in said second bin; and using said first and second average waveforms for suppressing said oscillatory signal Sosc from said composite signal S in the respective states of said first and second average waveforms.

In other words, an oscillatory signal Sosc can be suppressed from a composite signal S comprising the oscillatory signal Sosc and a modulating signal Smod without removing parts of the modulating signal Smod. The composite signal S is high pass filtered to produce estimates of oscillatory signal Sosc and the modulating signal Smod. The estimate of the oscillatory signal Sosc comprises at least first oscillations during a first state of the modulating signal Smod and second oscillations during a second state of the modulating signal Smod. A first bin associated with said first state and a second bin associated with said second state are defined and the first bin for said first oscillation according to a state defined from the estimate of the modulating signal Smod and the second bin for said second oscillation according to a state defined from the estimate of the modulating signal Smod are assigned. A first average waveform for said first oscillations in said first bin and a second average waveform for said second oscillations in said second bin are formed. And these first and second average waveforms are subtracted from the composite signal S in the respective states of said first and second average waveforms to form the modulating signal Smod. The method may be applied, for example, for suppressing the cardiogenic oscillations in an impedance pneumography signal, wherein the cardiogenic oscillations and the impedance respiratory signal form a transthoracic impedance signal.

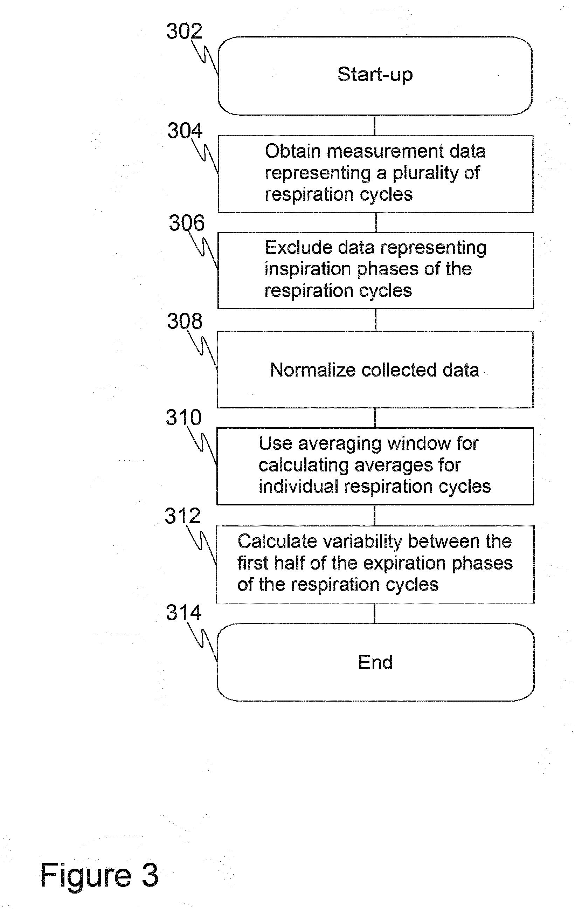

Fig. 3 depicts an embodiment of the method in accordance with the present invention.

At 302, a measuring means arrangement may be used or configured for collecting measurement data pertaining to respiratory measurements, or a source, such as a database, containing measurement data pertaining to respiratory measurements may be accessed for obtaining the measurement data.

At 304, respiratory measurement data representing a plurality of respiration cycles is obtained. Such respiratory data may comprise volume and respiratory flow measurement data representing flow and volume of respiration, or respiratory flow and time measurement data representing respiratory flow and time of respiration, or volume and time measurement data representing volume over a duration of time in respiration, wherein such data comprises measurement data of measurements from a plurality or respiration cycles and over a duration of time. The respiratory measurement data may comprise respiration cycles over a duration of time, such as at least several minutes, several hours, such as 5 hours or more, or the duration of night sleep, wherein the measurements of the respiration cycles over a duration of time are preferably successive and continuous at least in preferred windows of time, such as in specific sleep stages. The respiratory measurement data that is analyzed must only pertain to a single person and preferably the respiration cycles comprise of successive respiration cycles over a continuous period of time, such as respiration cycles over a one night's sleep or other such sufficient time duration during sleep at any time of the day. Additionally, respiratory measurements pertaining to respiration cycles during different sleep stages may be used and using respiratory measurement data pertaining to a plurality of respiration cycles over a plurality of sleep stages may yield a more robust quality of data for the method. Alternatively, respiratory measurements pertaining to preferred one or more particular sleep stages may be obtained and used. Alternatively, the respiratory measurement data may also pertain to successive respiration cycles over a continuous period of time, during a non-sleep stage, such as during state of wakefulness. Different persons or even the same person at different times may have different variability in their tidal breathing and therefore the present method is preferably conducted with respiratory measurement data pertaining to respiration measurement conducted to a person in a timeframe, such that the measurement is essentially continuous or such that the respiration cycles comprise time-wise successive respiration cycles.

Although respiration measurement is commonly mentioned herein, the respiratory measurement data may be obtained from measurement data pertaining to only the expiration phases of the respiration cycles.

The obtaining of respiratory measurement data may also comprise a threshold for the amount of respiration cycle measurements required, i.e. amount measurement data and/or sufficient timespan of respiration cycle measurements, which is required for the respiratory measurement data to be allowed or deemed sufficient to be analyzed with the method. For example, such a threshold may comprise TB expiration phase flow-volume, flow-time or time-volume measurements of the respiration cycles over a duration of time of at least 5 hours. An increased amount of measurement data may increase the accuracy of analyzing the variability in tidal breathing over time but a person skilled in the art will understand that the sufficiency of data as well as the type of data (e.g. whether pertaining to respiration cycles during a number of sleep stages and/or wake state) may vary e.g. in view of the application of the method and the quality or type of the measurement data and even the desired accuracy of the method.

The respiratory measurement data of TB expiration phase flow-volume, flow-time or time-volume measurements of the respiration cycles may be measured with impedance pneumoghraphy measuring means as described hereinbefore. Some other feasible measuring means and techniques for measuring respiratory volume, time and/or flow data comprise sensor measurement arrangements arranged to bed, mattress, blanket, and the like, which are usually based on capacitive measurements, such as ballistocardiography. Some further feasible measurement arrangements comprise wearable devices, such as clothes or straps measuring stretch, one example including Respiratory Inductive Plethysmography (RIP). Further, Doppler radar sensors arrangement (e.g. discussed in DOI: 10.1109/TMTT.2013.2256924), Optoelectrical pleythosmography (e.g. by PneumaCare), Electromagnetic induction plethysmography (e.g. by VoluSense) and accelerometer based arrangements may be used. Clearly, also other suitable means for acquiring flow-volume, flow-time or time-volume measurements of respiration cycles from tidal breathing may be used.

The measurement data comprises preferably TB respiration cycle measurements conducted to a single person at rest, such as to a sleeping person whether at night or during the day. The method may be carried out to existing data sets, such as by obtaining the measurement data for the use of the method from a database, cloud or other such source. Hence, the method needn't comprise conducting the actual measurement for collecting the measurement data. Also, respiratory measurement data may be obtained from a data set pertaining to the measurement of only the expiration phases of a person. Clearly, also respiratory measurement data pertaining to a plurality of respiration cycles may be obtained from a dataset representing respiratory measurement data of a plurality of persons whereat the data is filtered such that relevant respiration cycle data pertaining to only a single person is selected.

The measurement data may be also preprocessed or processed at this point for example in view of signal filtering for example to remove cardiogenic oscillations from the composite signal of the flow-volume, flow-time or time-volume respiration measurements. The measurement data may be also preprocessed or processed at this point to discard sections of data distorted by motion, talking, crying, cough, etc, which may have incurred during measurement. Further, the measurement data may also be processed or preprocessed to improve the measurement accuracy by applying one or more calibration coefficients or calibration models to the composite signal or filtered signal of the flow-volume, flow-time or time-volume respiration measurement.

At 306, the data representing the inspiration phases of the respiration cycles may be excluded. This method item isn't mandatory in case the measurement data comprises only measurement data of expiration phases e.g. when inspiration phases have not been measured, have been omitted or when the respiratory measurement data has been provided for the method such that the respiratory measurement data only comprises measurement data of expiration phases.

At 308, the measurement data may be normalized so that the expiration volume or time is normalized to a constant range, such as to 0-100%. Further, the measurement data is normalized so that the flow of expiration is normalized such that the time-integral of expired flow equals that of the expired volume. Optionally, the measurement data may already be in a normalized form in which case this method item isn't mandatory. However, normalization of data is not mandatory and measurement data may also comprise measurement data that does not pertain to absolute measurements of volume of respiration or expired flow of air from the lungs. The measurement data may be in a relative form, e.g. as t_{ptef}D/tₑ (t_{ptef} = time to peak tidal expiratory flow, tₑ = total duration of expiration) ratio, which is calculated from measured flow and time of respiration, or as V_{ptef}/Vₑ(V_{ptef} = volume at peak tidal expiratory flow, Vₑ = volume at peak tidal expiratory flow) ratio, which is calculated from measured flow and volume of respiration. Measuring ratios t_{ptef}/tₑ and V_{ptef}/Vₑ have been discussed in prior art, e.g. in publication "An Official American Thoracic Society/European Respiratory Society Statement: Pulmonary Function Testing in Preschool Children." American Journal of Respiratory and Critical Care Medicine, 175(12), pp. 1304-1345.

At 310, respiration cycles and the expiration phases thereof may be calculated averages using moving averaging window. This is an optional method item but it has the benefit of making the calculation of the correlations between the respiration cycles more efficient since the correlations may be calculated from a plurality of averaged respiration cycles rather than from all the individual respiration cycles, which individual respiration cycles may be much larger in number. An example of an averaging scheme may comprise calculating the average of 20 successive individual respiration cycles and representing them as one averaged respiration cycle.

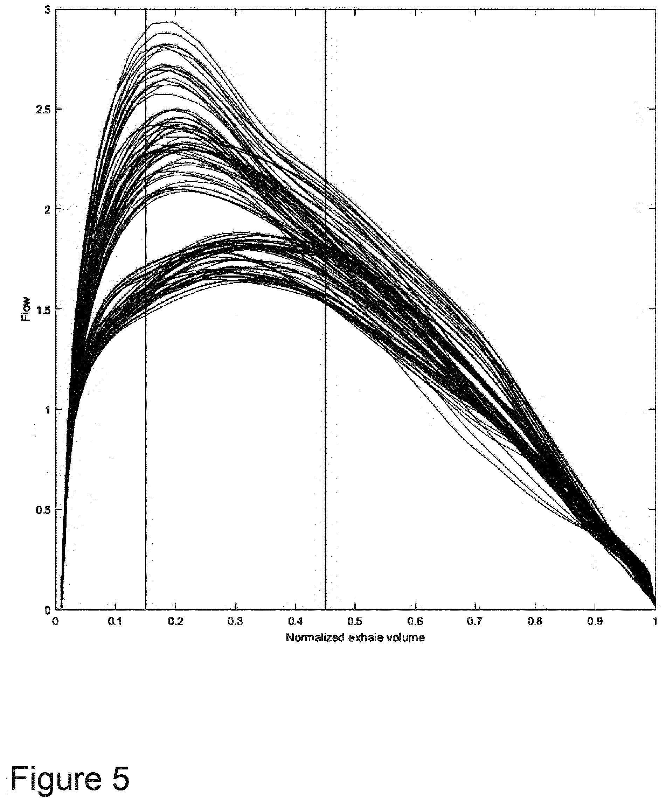

At 312, variability of the first half of the exhaled volume of the expiration phases between the individual or averaged respiration cycles over time is calculated. The variability may be calculated e.g. from correlations between the individual or averaged expiration phases of flow-volume, flow-time or time-volume measurements of respiration cycles representing respiration cycles over a duration of time. Also, other means for calculating variability between the individual and/or averaged expiration phases of flow-volume, flow-time or time-volume measurements of respiration cycles may be used. Examples of variability between averaged expiration phases of the respiration cycles in a flow-volume scale may be seen in Figs. 5-7.

At 314, the calculated correlations and/or calculated variability may be used to determine variability in expiration during tidal breathing. The level of variability in the first half of expired volume of expiration phase has been shown to be associated with the presence of airway obstruction, such as that lower level of variability in expiration phases indicates of the presence of some airway obstruction whereas higher level of variability in expiration phases indicates of healthy tidal breathing. Consequently, this may be used as a basis for lung disease diagnosis such as diagnosing asthma. Similarly, the determined level of variability in expiration phases may be used to determine drug or treatment efficiency. Clearly, this method step isn't mandatory to the method but it provides an example of a number of practical applications for the present invention.

The method of the present invention is preferably a computer-implemented method, which may be carried out on a computer, computer network or the like computing means. The arrangement of the present invention may use the impedance pneumography measuring means in accordance with Figs. 1 or 2, or other such described measuring means for measuring flow and volume of respiration or flow and time of respiration or time and volume of respiration over a duration of time and use computing means, such as a computer, computer network, or the like, at least functionally connected to the measuring means to collect measurement data from the measuring means and to execute analyzing of variability of the expiration phases of flow-volume, flow-time or time-volume measurements of the respiration cycles, wherein the variability between the expiration phases of the respiration cycles is analyzed from the measurement data in the range of the first half of expired volume in the expiration phase of the respiration cycles. Signal analysis as discussed may be also carried out on the computing means, computer network, or the like.

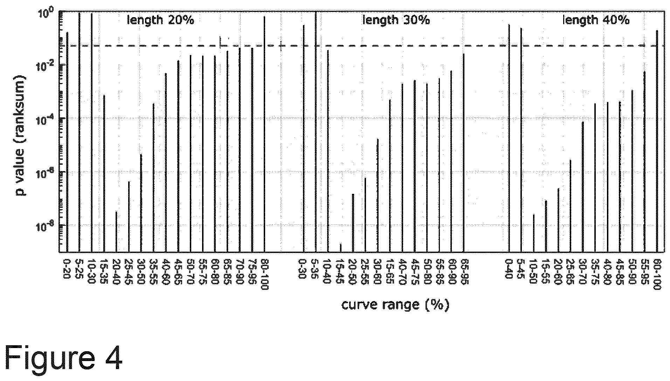

Fig. 4 depicts a diagram illustrating p-values of the comparison between the measurements for a sample of two groups. The first group comprises 70 patients aged 2.5 (0.-5.7 median and range) years with at least 3 acute physician-witnessed lower airway obstructions, wherefrom a sample of 60 measurements of the group's persons who had been off ICS medication for 4 weeks was acquired. The second group to which the sample of the first group is compared against comprises 39 healthy control persons aged 4.3 (1.5-6.0 median and range) years who were measured for a total of 80 times. Linear correlations were calculated between all TB flow-volume measurements for different ranges. Variability was assessed as the interquartile range (r15-45IQR) of the correlation values for each overnight recording. The p-values were calculated using Wilcoxon rank-sum test between the two groups. From the different ranges calculated herein it is clear that the variability in correlation of the measurements in the range of 15-45% gives a significant indication of a difference between the measurements of the sample of the first group and the second group as indicated by the p-values.

The measurements comprise TBFV measurements taken from a plurality of persons over time during their sleep regardless of the sleep stage. As noted the measurements in the range of 15-45% of the exhaled volume gives the best indication of the differences between group of healthy persons and group of asthmatic persons but significant differences may be also found for ranges of 10-50% or 20-40%. Hence, the first half of volume or time of expiration of the respiratory cycles may refer also to other ranges wherein the maximum of the range is not over about 60%.

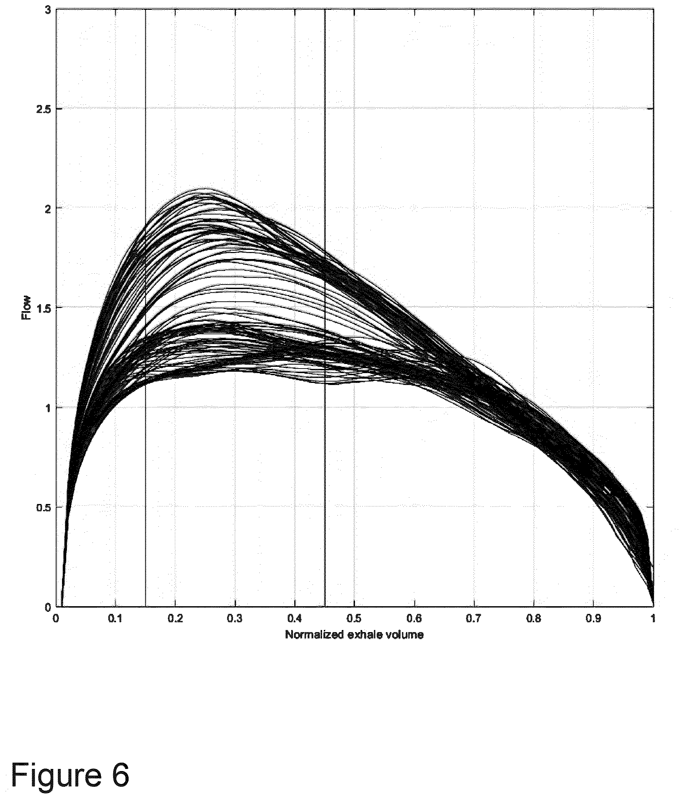

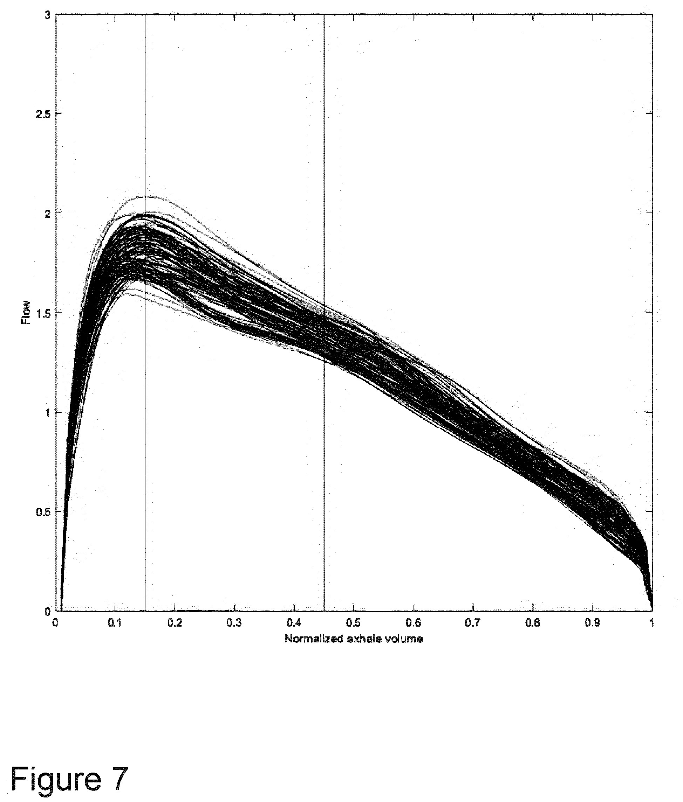

Clinical experimental evidence shows that the variability that is inherently present in tidal breathing is reduced in presence of obstructive airway diseases such as asthma or chronic obstructive pulmonary disease (COPD). This change stems from the response of the respiratory neural control centres as they integrate complex sensory information (lung baroreceptors, chemoreceptors, etc.) modulated by difficulty in breathing. As shown in figure 7 the small amount of variability in expiration phases in presence of asthma is clearly more pronounced in the early part of the expiratory flow-volume curve than in the latter part when compared to the early part of the expiratory flow-volume curves of figures 5 and 6. This is likely due to the fact that the activation of inspiratory muscles (diaphragm, intercostals) does not end sharply when expiration begins. Instead their activity continues and diminishes during the first part of expiration until expiration becomes completely passive only driven by the mechanical recoil of the lungs and the thorax in the latter part of expiration. This means that the early expiration is affected by respiratory neural control which is sensitive to airway obstruction and thus early expiration is better for assessing tidal breathing variability when aiming to detect presence of airway obstruction.

Figs. 5-7 illustrates TB measurements taken from a number of persons during their sleep regardless of the sleep stage and presented as flow-volume curves. The measurement data could also be presented as flow-time or volume-time curves. For clarity, the depicted expiration phase curves of the respiration cycles comprise averaged expiration phases of the respiration cycles.

In the figures, a preferred range of 15-45% of the first half, i.a. 15-45% of the expired volume of expiration phase of the respiration cycles is marked with two vertical lines to highlight the relatively high amount of variability in the respiration cycles in that range when compared to the rest of expiration phase.

Fig. 5 depicts a graph illustrating a plurality of flow-volume curves of expiration (i.e. excluding inspiration) obtained from a single person during a continuous measurement during sleep in a timeframe. In this case the sample comprises an asthmatic person on Inhaled Corticosteroid (ICS) medication. From the data a considerable variability in expiration over time may be detected in the range of the first half of expired volume of expiration.

Fig. 6 depicts another graph illustrating a plurality of flow-volume curves of expiration (excluding inspiration) obtained from a single person during a continuous measurement during sleep in a timeframe. In this case the sample comprises a person healthy from lung diseases. From the data a considerable variability in expiration over time may be detected in the range of the first half of expired volume of expiration.

Fig. 7 depicts another graph illustrating a plurality of flow-volume curves of expiration (excluding inspiration) obtained from a single person during a continuous measurement during sleep in a timeframe. In this case the sample comprises an asthmatic person who has been off of ICS medication for 4 weeks. From the data very little variation in expiration over time may be detected in the range of the first half of expired volume of expiration.

The scope of the invention is determined by the attached claims. The skilled persons will again appreciate the fact that the disclosed embodiments were constructed for illustrative purposes only, and the innovative fulcrum reviewed herein will cover further embodiments.

## Claims

1. A computer-implemented method for measuring changes in respiration using measurement data representing a plurality of measured respiration cycles in the form of flow and volume of respiration, or flow and time of respiration, or time and volume of respiration, over a duration of time, and wherein such measurement data pertains to at least the expiration phase measurement of the respiration cycles,
analyzing variability (312) of the expiration phases of flow-volume, flow-time or time-volume measurements of the respiration cycles wherein the measurements are measurements over a duration of time,
wherein the variability between the expiration phases of the respiration cycles is analyzed from the measurement data in the range of the first half of expired volume in the expiration phase of the respiration cycles.

2. The method of any preceding claim, wherein the measured respiratory cycles are analyzed from the measurement data in the range of 15-45% of expired volume in the expiration phase of the respiration cycle.

3. The method of any preceding claim, wherein the measured respiratory cycles are analyzed from the measurement data in the range of 10-50% of expired volume in the expiration phase of the respiration cycle.

4. The method of any preceding claim, wherein the respiration cycles of the measurement data are averaged over time using moving averaging window.

5. The method of any preceding claim, wherein the method comprises signal processing for removing cardiogenic oscillations from a number of measurement signals of the measurement data.

6. The method of any preceding claim, wherein the measurement data is processed to discard sections of data distorted by motion, talking, crying, or cough, or the like.

7. The method of any preceding claim, wherein the method comprises improving the measurement accuracy by applying one or more calibration coefficients or calibration models to a number of measurement signals of the measurement data.

8. The method of any preceding claim, wherein the respiration cycles comprise successive respiration cycles over a duration of time.

9. The method of any preceding claim, wherein the respiration cycles representing flow and volume of respiration or flow and time of respiration or time and volume of respiration pertain to measurements from continuous respiration measurement.

10. The method of any preceding claim, wherein the duration of time comprises at least several minutes, several hours or the duration of night sleep.

11. The method of any preceding claim, wherein the measurement data is normalized so that the expiration volume or time is normalized to a constant range, such as to 0-100%.

12. The method of any preceding claim, wherein the measurement data is normalized so that the expired flow is normalized such that the time-integral of expired flow equals that of the expired volume.

13. An arrangement for measuring changes in respiration comprising measuring means (30) for measuring flow and volume of respiration, or flow and time of respiration, or time and volume of respiration, over a duration of time, and wherein such measurement pertains to at least the expiration phase measurement of the respiration cycles,
and further comprising computing means arranged to,
analyze variability (312) of the expiration phases of flow-volume, flow-time or time-volume measurements of the respiration cycles wherein the measurements are measurements measured over a duration of time,
wherein the variability between the expiration phases of the respiration cycles is analyzed from the measurement data in the range of the first half of expired volume in the expiration phase of the respiration cycles.

14. The arrangement of claim 13, wherein measuring means (30) comprise impedance pneumography means.

15. The arrangement of claim 14, wherein the impedance pneumography means comprise using at least one electrode configured to be in contact with an arm of a human body and at least one electrode configured to be in skin contact with the thorax of the human body, and defining impedance signal changes which relate to the respiratory volume changes or time-differentiated impedance signal changes which relate to the respiratory flow.

16. A computer program product embodied in a non-transitory computer readable medium, comprising computer code for causing the computer to execute the method of claim 1.

## Patentansprüche

1. Computerimplementiertes Verfahren zum Messen von Änderungen in der Atmung unter Verwendung von Messdaten, die mehrere gemessene Atemzyklen in der Form von Atemfluss und -volumen oder Atemfluss und -zeit oder Atemzeit und -volumen über eine Zeitdauer repräsentieren, und wobei diese Messdaten wenigstens die Messung der Ausatmungsphase der Atemzyklen betreffen,
und Analysieren der Variabilität (312) der Ausatmungsphasen von Fluss-Volumen-, Fluss-Zeit- oder Zeit-Volumen-Messungen der Atemzyklen, wobei die Messungen Messungen über eine Zeitdauer sind,
wobei die Variabilität zwischen den Ausatmungsphasen der Atemzyklen anhand der Messdaten im Bereich der ersten Hälfte des ausgeatmeten Volumens in der Ausatmungsphase der Atemzyklen analysiert wird.

2. Verfahren nach einem der vorhergehenden Ansprüche, wobei die gemessenen Atemzyklen anhand der Messdaten im Bereich von 15-45 % des ausgeatmeten Volumens in der Ausatmungsphase des Atemzyklus analysiert werden.

3. Verfahren nach einem der vorhergehenden Ansprüche, wobei die gemessenen Atemzyklen anhand der Messdaten im Bereich von 10-50 % des ausgeatmeten Volumens in der Ausatmungsphase des Atemzyklus analysiert werden.

4. Verfahren nach einem der vorhergehenden Ansprüche, wobei die Atemzyklen der Messdaten unter Verwendung eines gleitenden Mittelungsfensters über die Zeit gemittelt werden.

5. Verfahren nach einem der vorhergehenden Ansprüche, wobei das Verfahren eine Signalverarbeitung zum Entfernen kardiogener Oszillationen aus einer Anzahl von Messsignalen der Messdaten umfasst.

6. Verfahren nach einem der vorhergehenden Ansprüche, wobei die Messdaten verarbeitet werden, um Datenabschnitte zu verwerfen, die durch Bewegung, Sprechen, Weinen oder Husten oder dergleichen verfälscht sind.

7. Verfahren nach einem der vorhergehenden Ansprüche, wobei das Verfahren das Verbessern der Messgenauigkeit durch Anwenden eines oder mehrerer Kalibrierungskoeffizienten oder Kalibrierungsmodelle auf eine Anzahl von Messsignalen der Messdaten umfasst.

8. Verfahren nach einem der vorhergehenden Ansprüche, wobei die Atemzyklen aufeinander folgende Atemzyklen über eine Zeitdauer umfassen.

9. Verfahren nach einem der vorhergehenden Ansprüche, wobei die Atemzyklen, die Atemfluss und -volumen oder Atemfluss und -zeit oder Atemzeit und -volumen repräsentieren, Messungen aus einer kontinuierlichen Atemmessung betreffen.

10. Verfahren nach einem der vorhergehenden Ansprüche, wobei die Zeitdauer mindestens mehrere Minuten, mehrere Stunden oder die Dauer des Nachtschlafes umfasst.

11. Verfahren nach einem der vorhergehenden Ansprüche, wobei die Messdaten normalisiert werden, so dass das Ausatmungsvolumen oder die Ausatmungszeit bezüglich eines konstanten Bereichs normalisiert ist, wie etwa 0-100 %.

12. Verfahren nach einem der vorhergehenden Ansprüche, wobei die Messdaten normalisiert werden, so dass der ausgeatmete Fluss so normalisiert wird, dass das Zeitintegral des ausgeatmeten Flusses gleich dem ausgeatmeten Volumen ist.

13. Anordnung zum Messen von Änderungen in der Atmung, welche Messmittel (30) zum Messen von Atemfluss und -volumen oder Atemfluss und -zeit oder Atemzeit und -volumen über eine Zeitdauer umfasst, und wobei diese Messung wenigstens die Messung der Ausatmungsphase der Atemzyklen betrifft,
und ferner Rechenmittel umfasst, die dazu eingerichtet sind, die Variabilität (312) der Ausatmungsphasen von Fluss-Volumen-, Fluss-Zeit- oder Zeit-Volumen-Messungen der Atemzyklen zu analysieren, wobei die Messungen Messungen sind, die über eine Zeitdauer ausgeführt werden,
wobei die Variabilität zwischen den Ausatmungsphasen der Atemzyklen anhand der Messdaten im Bereich der ersten Hälfte des ausgeatmeten Volumens in der Ausatmungsphase der Atemzyklen analysiert wird.

14. Anordnung nach Anspruch 13, wobei die Messmittel (30) Mittel zur Impedanzpneumographie umfassen.

15. Anordnung nach Anspruch 14, wobei die Mittel zur Impedanzpneumographie das Verwenden mindestens einer Elektrode, die dafür ausgelegt ist, sich mit einem Arm eines menschlichen Körpers in Kontakt zu befinden, und mindestens einer Elektrode, die dafür ausgelegt ist, sich in Hautkontakt mit dem Thorax des menschlichen Körpers zu befinden, und das Definieren von Änderungen des Impedanzsignals, welche mit Änderungen des Atemvolumens zusammenhängen, oder von Änderungen des nach der Zeit differenzierten Impedanzsignals, welche mit dem Atemfluss zusammenhängen, umfassen.

16. Computerprogrammprodukt, welches in einem nichtflüchtigen, computerlesbaren Medium verkörpert ist und Computercode zum Veranlassen des Computers, das Verfahren nach Anspruch 1 auszuführen, umfasst.

## Revendications

1. Procédé implémenté sur ordinateur pour mesurer des changements dans la respiration utilisant des données de mesure représentant une pluralité de cycles respiratoires mesurés sous la forme du débit et du volume de respiration, ou du débit et du temps de respiration, ou du temps et du volume de respiration, sur une durée, et dans lequel de telles données de mesure concernent au moins la mesure de phase expiratoire des cycles respiratoires,
d'analyser la variabilité (312) des phases d'expiration de mesures de débit-volume, débit-temps ou temps-volume des cycles respiratoires, dans lequel les mesures sont des mesures sur une durée,
dans lequel la variabilité entre les phases d'expiration des cycles respiratoires est analysée à partir des données de mesure dans la plage de la première moitié de volume expiré dans la phase expiratoire des cycles respiratoires.

2. Procédé selon l'une quelconque revendication précédente, dans lequel les cycles respiratoires mesurés sont analysés à partir des données de mesure dans la plage de 15-45 % du volume expiré dans la phase expiratoire du cycle respiratoire.

3. Procédé selon l'une quelconque revendication précédente, dans lequel les cycles respiratoires mesurés sont analysés à partir des données de mesure dans la plage de 10-50 % du volume expiré dans la phase expiratoire du cycle respiratoire.

4. Procédé selon l'une quelconque revendication précédente, dans lequel les cycles respiratoires des données de mesure sont moyennés sur la durée en utilisant une fenêtre de moyennisation mobile.

5. Procédé selon l'une quelconque revendication précédente, dans lequel le procédé comprend un traitement de signal pour retirer des oscillations cardiogènes d'un nombre de signaux de mesure des données de mesure.

6. Procédé selon l'une quelconque revendication précédente, dans lequel les données de mesure sont traitées pour rejeter des sections de données déformées par le mouvement, la parole, les pleurs ou la toux, ou similaire.

7. Procédé selon l'une quelconque revendication précédente, dans lequel le procédé comprend d'améliorer la précision de mesure en appliquant un ou plusieurs coefficients d'étalonnage ou modèles d'étalonnage à un nombre de signaux de mesure des données de mesure.

8. Procédé selon l'une quelconque revendication précédente, dans lequel les cycles respiratoires comprennent des cycles respiratoires successifs sur une durée.

9. Procédé selon l'une quelconque revendication précédente, dans lequel les cycles respiratoires représentant le débit et le volume de respiration ou le débit et le temps de respiration ou le temps et le volume de respiration concernent des mesures venant de mesure de respiration continue.

10. Procédé selon l'une quelconque revendication précédente, dans lequel la durée comprend au moins plusieurs minutes, plusieurs heures ou la durée d'une nuit de sommeil.

11. Procédé selon l'une quelconque revendication précédente, dans lequel les données de mesure sont normalisées de façon à ce que le volume ou le temps d'expiration soit normalisé à une plage constante, telle que 0-100 %.

12. Procédé selon l'une quelconque revendication précédente, dans lequel les données de mesure sont normalisées de façon à ce que le débit expiré soit normalisé de telle façon que l'intégrale de temps du débit expiré soit égale à celle du volume expiré.

13. Agencement pour mesurer des changements dans la respiration, comprenant des moyens de mesure (30) pour mesurer le débit et le volume de respiration ou le débit et le temps de respiration ou le temps et le volume de respiration, sur une durée, et dans lequel de telles données de mesure concernent au moins la mesure de phase expiratoire des cycles respiratoires
et comprenant en outre des moyens de calcul agencés pour :
analyser la variabilité (312) des phases d'expiration de mesures de débit-volume, débit-temps ou temps-volume des cycles respiratoires, dans lequel les mesures sont des mesures mesurées sur une durée,
dans lequel la variabilité entre les phases d'expiration des cycles respiratoires est analysée à partir des données de mesure dans la plage de la première moitié de volume expiré dans la phase expiratoire des cycles respiratoires.

14. Agencement selon la revendication 13, dans lequel les moyens de mesure (30) comprennent des moyens de pneumographie d'impédance.

15. Agencement selon la revendication 14, dans lequel les moyens de pneumographie d'impédance comprennent d'utiliser au moins une électrode configurée pour être en contact avec un bras d'un corps humain et au moins une électrode configurée pour être en contact de la peau avec le thorax d'un corps humain, et de définir des changements de signal d'impédance qui sont liés aux changements de volume respiratoire ou à des changements de signal d'impédance différenciés dans le temps qui sont liés au flux respiratoire.

16. Produit de programme informatique dans un support nontransitoire lisible sur ordinateur, comprend un code informatique pour que l'ordinateur exécute le procédé selon la revendication 1.
